# EUROPÄISCHE PATENTSCHRIFT

(11) **EP 0 436 154 B1**
(45) Veröffentlichungstag und Bekanntmachung des Hinweises auf die Patenterteilung: **15.09.1993**
(21) Anmeldenummer: 90123771.9
(22) Anmeldetag: 11.12.1990
(51) Int. Cl.: A61M 5/00, A61M 25/00, A61M 1/28

(54) **Transcutaner Implantatkatheter**
Transcutaneous implantation catheter
Cathéter d'implantation transdermique

(30) Priorität: 30.12.1989 DE 3943412
(43) Veröffentlichungstag der Anmeldung: 10.07.1991
(73) Patentinhaber: B. Braun Melsungen AG, 34209 Melsungen (DE)
(72) Erfinder: Verreet, Patrick, Dr., W-4005 Meerbusch 1 (DE); Haacke, Claus, Dr., W-3508 Melsungen (DE)
(74) Vertreter: Selting, Günther, Dipl.-Ing.

(56) Entgegenhaltungen:
- EP-A- 0 102 342
- EP-A- 0 164 896
- US-A- 3 633 585
- US-A- 4 278 092
- US-A- 4 392 855
- DER CHIRURG, 54. Jahrgang, 1983, Springer-Verlag, Berlin, Heidelberg P.R. VERREET et al. "Implantationstechnik des Oreopoulos-Zellermann-Katheters zur Peritonealdialyse" Seiten 609-612

## Beschreibung

Die Erfindung betrifft einen transcutanen Implantatkatheter der im Oberbegriff des Patentanspruchs 1 angegebenen Art. Neben dem Zugang zu Blutgefäßen und anderen Hohlräumen des menschlichen Körpers ist ein Einsatzgebiet die Peritonealdialyse.

Für die kontinuierliche ambulante Peritonealdialyse (CAPD) werden Peritoneal-Katheter benötigt, die durch die Bauchdecke des Patienten hindurchführen und in die Bauchdecke einwachsen. Aus "Der Chirurg" (1983) 609-612 ist der o.s. Peritoneal-Katheter bekannt, der aus einem durchgehenden Schlauch mit drei Abschnitten besteht. Zwischen dem intraperitonealen ersten Abschnitt und dem zum Einwachsen in die Bauchdecke bestimmten zweiten Abschnitt befinden sich Halteorgane in Form einer Muffe zur Begrenzung des Bauchwandabschnittes, einer Dacron-Scheibe und eines Silastic-Balls zur Fixierung des Katheters an dem hinteren Blatt der Rectus-Scheide. Zwischen dem zweiten Abschnitt und dem extracorporalen dritten Abschnitt ist eine Dacron-Muffe zur Verankerung in der Subcutis vorgesehen. Der bekannte Peritoneal-Katheter besteht insgesamt aus einem außen glatten Silikon-Schlauch. Der zum Einlegen in das Muskel- bzw. Hautgewebe bestimmte zweite Abschnitt bewirkt im Implantationstunnel des Gewebes die Ausbildung einer glatten, nicht auf dem Katheter haftenden Umscheidung zwischen den aus Manschette und Scheibe bestehenden Verwachsungsstellen. Im Spalt zwischen Katheter und Umscheidung sammelt sich Flüssigkeit, welche sich leicht infizieren kann. Außerdem ist festgestellt worden, daß dieses Gebiet aufgrund der verwendeten Dacron-Manschetten in der Haut-Einwachszone dauernd einer starken Reaktion zwischen Fibroblasten, Makrophagen und Hautzellen ausgesetzt ist und sich daher häufig entzündet.

Bekannt sind ferner perkutane Zugangseinrichtungen, die mit dem Körpergewebe verwachsen und eine Zugangsstelle für einen einzuführenden Katheter bilden. Eine solche Zugangseinrichtung ist in EP-A-0 164 896 (Thermedics) beschrieben. Der in Verbindung mit der Zugangseinrichtung benutzte Katheter ist ebenfalls außen glatt, so daß das Körpergewebe im Implantationstunnel eine den Katheter umgebende Umscheidung bildet.

DE-A-28 06 030 und DE-A-33 45 513 beschreiben Gefäßprothesen aus einem porösen Schlauch, der aus einem Faservlies aus feinen Polyurethan-Fasern besteht. An derartige Gefäßprothesen wächst das Körpergewebe an. Gefäßprothesen ersetzen Blut- oder andere Körpergefäße, die z.B. aufgrund von Thrombosen entfernt werden mußten. Demgemäß werden Gefäßprothesen vollständig in den Körper des Patienten eingesetzt. Die in EP 0 164 896 beschriebene Zugangseinrichtung besitzt zwei unterschiedlich poröse Vlieslagen aus PUR-Fasern, die an definierten Stellen bei der subcutanen Implantation das Einwachsen von Epithelzellen und kollagenproduzierenden Fibroblasten ermöglichen sollen. Die Vlieslage mit der engeren Porenweite ist auf der Oberfläche des "Halses" der Zugangseinrichtung angebracht und soll 75 - 100 µm Faserabstand besitzen. Sie wird zur Haut orientiert und soll das Einwachsen von Epithelzellen ermöglichen. Demgegenüber wird der scheibenförmige Teil der Zugangseinrichtung, der im Subcutanbereich liegt, mit einem Faserabstand von 400 - 800 µm definiert und soll das Einwandern von Fibroblasten, die dann Kollagenfasern bilden, ermöglichen.

Der Erfindung liegt die Aufgabe zugrunde, einen transcutanen Implantatkatheter zu schaffen, bei dem die Gefahr von Abwehrreaktionen und Infektionen im Implantationstunnel verringert ist. Weiterhin sollen die Zellen, die mit dem Katheter verwachsen sollen, die von ihnen benötigten Faserabstände auch dann vorfinden, wenn der Katheter nicht millimetergenau chirurgisch implantiert wurde, sondern z.B. mittels Stilett-Technik nur ungefähr plaziert wurde.

Die Lösung dieser Aufgabe erfolgt erfindungsgemäß mit den im Patentanspruch 1 angegebenen Merkmalen.

Bei dem erfindungsgemäßen transcutanen Katheter besteht die Außenfläche des zweiten (mittleren) Abschnittes aus einem porösen Faservlies, an das das Gewebe unmittelbar anwachsen kann. Das Anwachsen erfolgt also über die gesamte Länge des zweiten Abschnitts gleichmäßig, so daß eine vollständige Verwachsung mit dem zweiten Abschnitt entsteht. Das Körpergewebe wächst an den zweiten Abschnitt des Katheters an und umgibt diesen abdichtend. Wegen des geringen Fremdkörperreizes und wegen des Fehlens von Riesenzellen wandern bis zu mehreren Monaten nach einer Implantation langsam Fibroblasten in die Wand des zweiten Katheterabschnittes ein, die dann auch Kollagen produzieren. Kollagenfaserbündel verbinden den Katheter mit dem umgebenden Gewebe in Form einer Kapsel. Diese Kapsel ist leicht lösbar. Der Katheter wird somit über die gesamte Länge des zweiten Abschnitts in das Empfängergewebe integriert. Die Länge dieses zweiten Abschnittes beträgt etwa 5 cm. Der Innendurchmesser des zweiten Abschnittes beträgt vorzugsweise etwa 4 mm bei einer Wandstärke von etwa 1 mm.

Bei dem erfindungsgemäßen Katheter diffundieren Fibroblasten in das lockere Vlies ein, wodurch eine Kollagenmatrix entsteht, die die Verwachsung mit dem Stichkanal darstellt. Die Kollagenfibrillen stoppen die ungehemmte Wanderung von Hautepithelzellen, die sonst neben der Schlauchoberfläche nach unten wachsen würden, wodurch eine Tasche um den Katheter entsteht, der Katheter also ohne Verbindung zur Haut in einem Umscheidungskanal liegt. Zudem können die Hautepithelzellen zwischen die Vliesfibrillen einwandern, bis der Abstand zwischen den Fasern zu klein wird und damit das Einwachsen aufhört.

Wie Tierexperimente gezeigt haben, ist ein Vlies, dessen Faserabstand von der untersten bis zur obersten Lage gleichmäßig bei ca. 80 - 100 µ liegt, manchmal ungünstig. Dies kommt daher, daß wegen des geringen Faserabstandes die auf dem Faserwerk aufliegenden Fremdkörperriesenzellen eine komplette Abdichtung machen und dadurch keine Fibroblasten mehr einwandern können. Erst die graduelle Lockerung der Vliesstruktur - d.h. die Abstände zwischen den Fasern werden allmählich von innen nach außen größer - führt zu einer besseren Einwachsreaktion. Jetzt decken die Fremdkörperriesenzellen nicht mehr alle Lücken im Vlies ab, die Fibroblasten diffundieren ein und es entsteht in tieferen Schichten eine Kollagenverbindung. Im Hautniveau wandern Hautepithelzellen ein und verwachsen mit dem Vlies. Weil aber Hautzellen und Fibroblasten unterschiedlich groß sind (Hautepithelzellen ca. 5 - 10 µ, Fibroblasten 40 - 50 µ), sind in den verschiedenen Einwachsniveaus verschiedene Faserabstände zweckmäßig.

Soll jedoch der Katheter, wie sehr häufig durchgeführt, mit Seldinger-, Stilett- oder Braunülentechnik in einen Körperhohlraum verlegt werden, wobei ein Stück des Katheters unter der Haut durchgezogen (= getunnelt) wird, ist zu erwarten, daß die Zuordnung des "richtigen" Faserabstands im Vlies zum Gewebe nicht immer genau so sein wird, daß der Einwachsprozeß ordnungsgemäß stattfinden kann. Dies kann nur dann gewährleistet werden, wenn alle benötigten Faserabstände (geringe Abstände für Epithelzellen und größere Abstände für Fibroblasten) an allen Stellen der Tunnelingzone vorhanden sind.

Vorzugsweise besteht der dritte Abschnitt des Schlauches aus demselben Material wie der zweite Abschnitt, jedoch ist im dritten Abschnitt auf der Schlauchoberfläche eine geschlossene glatte Beschichtung vorgesehen. Die Beschichtung verhindert, daß Kontaminationen in den extracorporalen Katheterabschnitt eintreten können, und verleiht diesem Abschnitt auch eine größere Steifigkeit, verglichen mit dem zweiten Abschnitt. Der zweite Abschnitt ist weicher und biegsamer als bei den bekannten Kathetern, so daß das Biegen des Katheters im Implantationstunnel erleichtert wird und die auf die Wände des Tunnels einwirkenden Rückbiegekräfte verringert sind. Das Gewebe wird durch den weichen zweiten Abschnitt nicht irritiert. Der im Körperhohlraum, wie Blutgefäß, Peritoneum oder Blase liegende erste Abschnitt hat eine glatte geschlossene Oberfläche. Da dieser Abschnitt im Körperhohlraum liegt und nicht zum Einwachsen von Körpergewebe bestimmt ist, kann er etwas steifer sein als der zweite Abschnitt.

Der erfindungsgemäße transcutane Implantatkatheter eignet sich als Dauerkatheter, insbesondere für die kontinuierliche ambulante Peritonealdialyse (CAPD).

Im folgenden wird unter Bezugnahme auf die Zeichnungen ein Ausführungsbeispiel der Erfindung anhand eines Peritonealkatheters näher erläutert. Anstelle des Peritoneums können auch andere Körperhohlräume katheterisiert werden.

Es zeigen:
- Fig. 1: eine Ansicht des Peritonealkatheters,
- Fig. 2: eine Darstellung der Katheterlage nach Implantation,
- Fig. 3: eine Ansicht eines Stilettkatheters und
- Fig. 4: den Querschnitt der Vlieszone entlang der Linie IV-IV in Fig. 3.

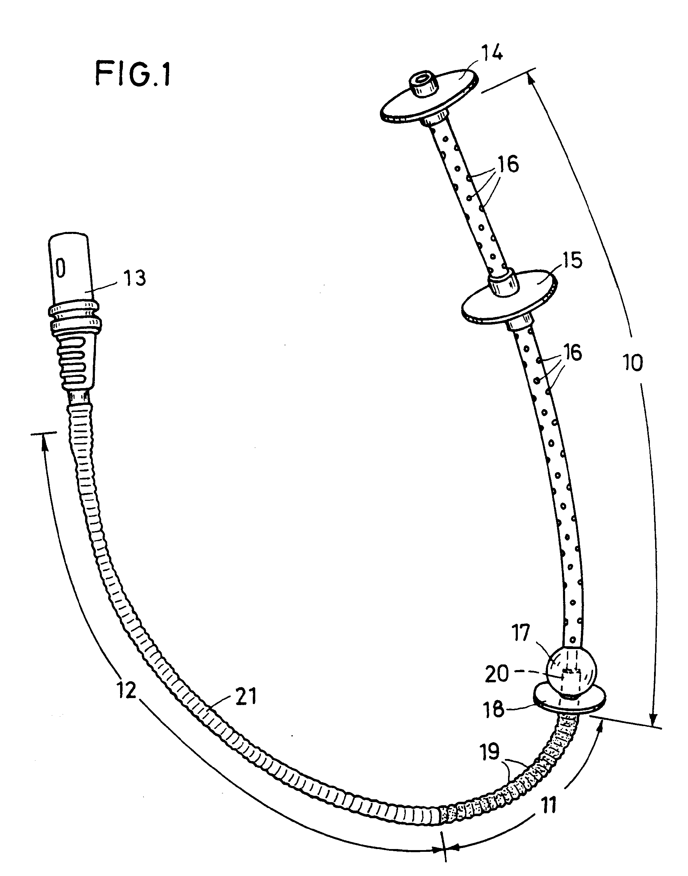

Der in Fig. 1 dargestellte Katheter besteht aus einem Schlauch, dessen Innendurchmesser über die gesamte Länge im wesentlichen konstant ist. Der Schlauch weist einen intraperitonealen ersten Abschnitt 10, einen zum Einwachsen in das Haut- und Muskelgewebe bestimmten zweiten Abschnitt 11 und einen extracorporalen dritten Abschnitt 12 auf. Am Ende des dritten Abschnitts 12 ist ein Konnektor 13 befestigt.

Der erste Abschnitt 10 ist in bekannter Weise mit zwei abstehenden Scheiben 14,15 versehen, die in Längsrichtung des Schlauches einen Abstand voneinander haben. Diese Rückhaltescheiben dienen dazu, den Katheter im Gebiet des Douglas'schen Raumes zurückzuhalten. Im ersten Abschnitt 10 sind zahlreiche Löcher 16 für Flüssigkeitszu- und -ablauf vorgesehen. Außerdem ist das eine Ende des Abschnitts 10 offen. An dem dem offenen Ende abgewandten Ende des Abschnitts 10 befindet sich eine Abdichtkugel 17, die unterhalb der hinteren Fascie des musculus rectus zu liegen kommt. Unmittelbar an die Abdichtkugel 17 schließt sich der zweite Abschnitt 11 an, der angrenzend an die Abdichtkugel 17 eine radial abstehende Scheibe 18 zum Einwachsen und Abdichten des Peritoneums aufweist.

Anstelle der Rückhaltescheiben können jedoch auch andere bei Kathetern bekannte Rückhaltevorrichtungen zur Verwendung kommen. Dies sind Pigtaileinrollungen, Malecot- oder Pezzerschirme sowie Ballons.

Ebenso können anstelle der Abdichtkugel und -scheibe eine Anschlagschulter oder ein aufsitzender Ring mit umlaufender Rille Verwendung finden.

Der zweite Abschnitt 11 besteht aus einer Hülse aus einem Faservlies aus dünnen Polyurethan-Fasern. Dieses Faservlies ist nach dem Verfahren der DE-A-28 06 030 durch Aufwickeln zahlreicher Fäden hergestellt. Diese dünnen Fäden, die eine Stärke von 4 - 5 µm haben, werden mittels Sprühtechnik erzeugt. Hierbei wird eine Polyurethan-Lösung aus einer Düse ausgeblasen und die dabei entstehenden Fäden werden auf eine Stange oder Welle aufgewickelt. Dabei entsteht eine unregelmäßige Netzstruktur aus Fäden, die an den Kreuzungspunkten aneinander haften. Der Faserabstand wächst dabei infolge der Wahl der Sprühbedingungen von unten nach oben. Er kann kontinuierlich oder in Stufen ansteigen. So ist zum Beispiel die unterste Schicht mit 40 - 60 µ Abstand, die mittlere mit 80 - 100 µ und die oberste mit 200 - 600 µ Faserabstand herzustellen. Ebenso sind andere Kombinationen und Abstufungen denkbar. Die Schichtdicken betragen jeweils etwa 0,7 - 0,5 mm. Zur Definition der Vliesporosität hat sich herausgestellt, daß die Durchströmungszeit mit einem Medium unter definierten Druckverhältnissen bezogen auf eine Einheitsfläche eine bessere Meßgröße darstellt als die Benennung von mittleren Faserabständen. In der so entstandenen Hülse ist zur Vermeidung von Einknickungen ein schraubenförmiger Verstärkungsfaden 19 vorhanden, wobei das Verfahren von DE-A-33 45 513 angewandt werden kann, damit die einzelnen Fadenwindungen den erforderlichen Abstand voneinander haben. Der Abschnitt 11 des Schlauchs ist offenporig fibrillär aufgebaut, d.h. die Vliesstruktur erstreckt sich über die gesamte Stärke der Schlauchwand.

Der Abschnitt 11, der eine Länge von etwa 5 cm und etwa den gleichen Außendurchmesser wie der erste Abschnitt 10 oder der dritte Abschnitt 12 hat, geht einstückig in den extracorporalen dritten Abschnitt 12 über, der aus demselben Material besteht wie der Abschnitt 11, jedoch außen mit einer dichten und glatten Beschichtung 21 versehen ist. Durch die Beschichtung 21 erhält der Abschnitt 12 eine etwas größere Steifigkeit als der sehr weiche und flexible zweite Abschnitt 11.

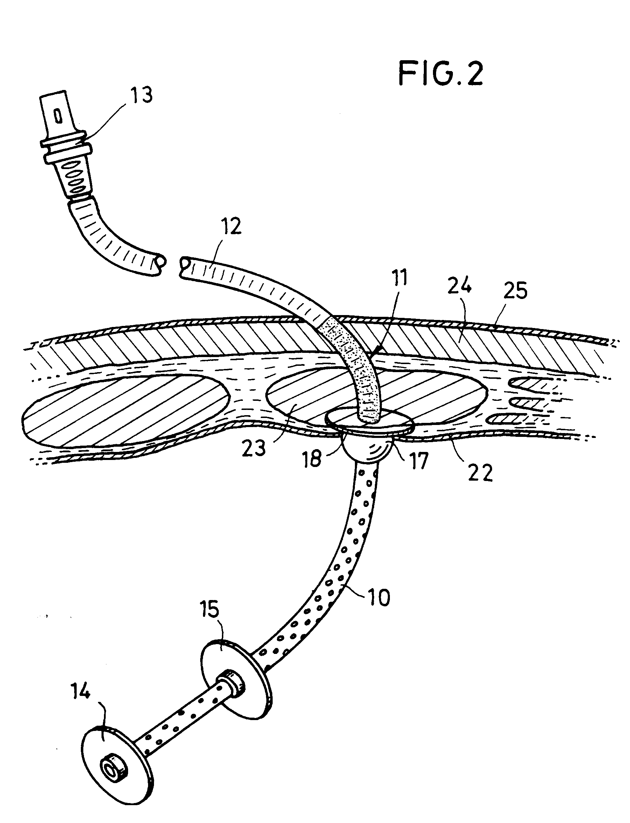

Fig. 2 zeigt den Katheter im implantierten Zustand, wobei erkennbar ist, daß die Abdichtkugel 17 sich unterhalb des Peritoneums 22 als Widerlager befindet. Nach dem Einführen der Kugel 17 ist die Öffnung des Peritoneums bei hindurchgehendem Katheter zugenäht worden. Der Abschnitt 11 führt durch den musculus rectus 23, wobei die Scheibe 18, die aus dem Fasermaterial des zweiten Abschnitts 11 besteht, auf dem Peritoneum 22 abgestützt ist und in das Gewebe einwachsen kann. Der zweite Abschnitt 11 erstreckt sich ferner durch die Subcutis und die Cutis 25. Außerhalb des Körpers liegt der dritte Bereich 12 des Katheters.

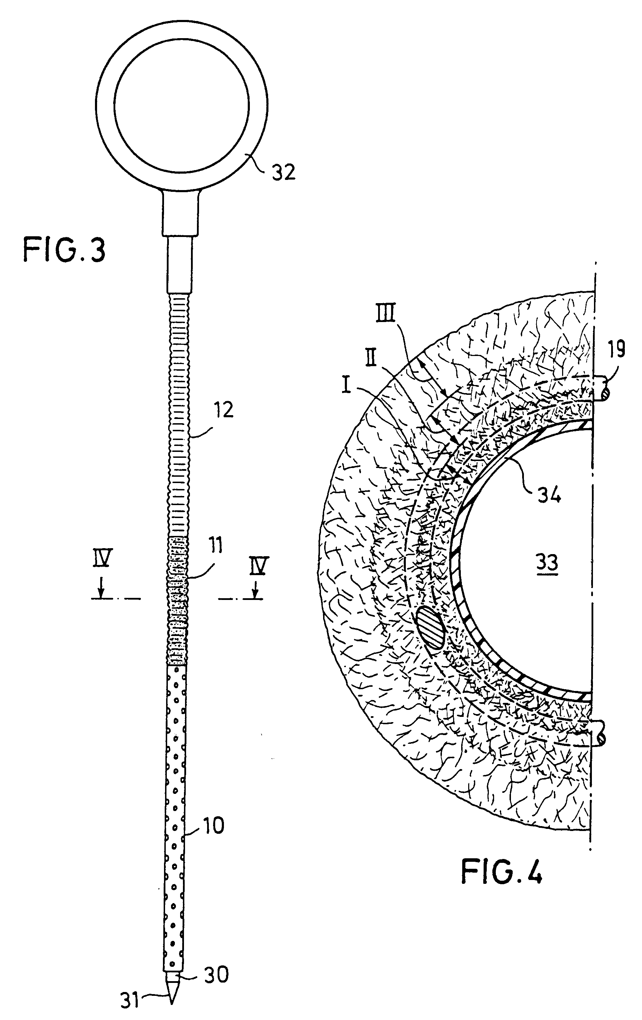

Fig. 3 zeigt einen Katheter, der die gleichen Abschnitte besitzt wie in Fig. 1 und Fig. 2, jedoch sind keine auf dem Katheter sitzende Scheiben und Kugeln vorhanden. Der Katheter befindet sich auf einem Stilett 30, dessen scharfe Spitze 31 aus dem Katheter herausragt und das am hinteren Ende einen Griff 32 aufweist. Der CAPD-Katheter wird mittels Punktion verlegt.

Fig. 4 zeigt einen Querschnitt von einem der Katheter nach den Fign. 1 bis 3 durch die Vlieszone 11, wie z.B. den Schnitt IV-IV von Fig. 3. Man erkennt das Innenlumen 33 des Katheters, welches durch eine flüssigkeitsundurchlässige Schicht 34 begrenzt ist, worüber sich die Vlieszonen I,II und III, die durch ansteigenden Faserabstand gekennzeichnet sind, anschließen.

Die unterste Vlieszone I besitzt Faserabstände von ca. 40 - 60 µ und ist ca. 0,2 mm dick, darüber liegt die Zone II mit Faserabständen von ca. 80 - 100 µ und einer Dicke von ca. 0,4 mm und als oberste Schicht liegt die Zone III mit 200 - 600 µ Faserabständen und einer Dicke von 0,4 mm. Die angegebenen Faserabstände sind jeweils die "mittleren Faserabstände".

Der zweite Abschnitt 11 enthält vorzugsweise einen schraubenförmig verlaufenden Verstärkungsstrang oder -draht 19. Diese schraubenförmige Verstärkung dient zur Verbesserung der Knickstabilität bei engen Biegeradien. Trotz erhöhter Knickstabilität ergibt sich eine niedrige radiale Rückstellkraft des Katheters im Tunnelingbereich, wodurch eine Nekrotisierung des umgebenden Gewebes verhindert wird. Die schraubenförmige Verstärkung ist in das Faservlies eingebettet. Die flüssigkeitsundurchlässige Schicht 34 (Fig. 4) bildet lediglich eine dünne Beschichtung an der Innenseite des rohrförmigen Faservlieses, jedoch keinen eigenständigen Schlauch. Sie hat also keine wesentliche Verstärkungsfunktion. Die Verstärkung wird überwiegend von dem schraubenförmigen Verstärkungsstrang ausgeübt, der so beschaffen ist, daß die Kompressibilität im Abschnitt 11 niedriger ist als in den anderen Abschnitten des Katheters. Auch die Rückstellfähigkeit ist im Abschnitt 11 niedriger als in den anderen Abschnitten des Katheters.

## Patentansprüche

1. Transcutaner Implantatkatheter, der durch eine Tunnelingzone in einen Körperhohlraum verlegt wird, bestehend aus einem Schlauch mit einem intracorporalen ersten Abschnitt (10) aus weichelastischem Vollkunststoff, einem zum Einlegen in das Tunnelgewebe bestimmten zweiten Abschnitt (11) und einem extracorporalen dritten Abschnitt (12),
**dadurch gekennzeichnet,**
daß der zweite Abschnitt (11) aus einem Faservlies mit offenporig fibrillärer Struktur besteht, wobei sich der mittlere Faserabstand im Faservlies von innen nach außen stufenartig oder kontinuierlich erhöht.

2. Transcutaner Implantatkatheter nach Anspruch 1, dadurch gekennzeichnet, daß der dritte Abschnitt (12) aus demselben Material wie der zweite Abschnitt (11) besteht und diesem einstückig angeformt ist und eine geschlossene glatte Beschichtung (21) aufweist.

3. Transcutaner Implantatkatheter nach Anspruch 1 oder 2, dadurch gekennzeichnet, daß am Übergang zwischen erstem und zweitem Abschnitt (10,11) ein Hilfsmittel wie Scheibe, Kugel, Ringnut oder Rille angebracht ist, welches das Annähen und Einwachsen an der Durchtrittsstelle in den Körperhohlraum zum Abdichten ermöglicht.

4. Transcutaner Implantatkatheter nach einem der Ansprüche 1-3, dadurch gekennzeichnet, daß der zweite Abschnitt (11) einen schraubenförmig verlaufenden Verstärkungsfaden (19) aufweist.

5. Transcutaner Implantatkatheter nach einem der Ansprüche 1-4, dadurch gekennzeichnet, daß die Fasern des Faservlieses eine Stärke von etwa 4 bis 5 µm haben.

6. Transcutaner Implantatkatheter nach einem der Ansprüche 1-5, dadurch gekennzeichnet, daß das Faservlies aus Polyurethan besteht.

7. Transcutaner Implantatkatheter nach einem der Ansprüche 1-6, dadurch gekennzeichnet, daß der zweite Abschnitt (11) weicher und biegsamer ist als der erste Abschnitt (10).

8. Transcutaner Implantatkatheter nach einem der Ansprüche 1-7, dadurch gekennzeichnet, daß die Länge des zweiten Abschnitts (11) etwa 5 cm beträgt.

9. Transcutaner Implantatkatheter nach einem der Ansprüche 1-8, dadurch gekennzeichnet, daß der mittlere Faserabstand im Faservlies von innen nach außen im Bereich von 40 µm bis 600 µm anwächst.

## Revendications

1. Cathéter d'implantation transdermique, posé dans une zone de tunneling, dans une cavité corporelle, composé d'un tuyau souple avec une première section (10) intracorporelle, en matériau totalement synthétique doté d'une élasticité molle, une deuxième section (11) conçue pour l'insertion dans le tissu du tunnel et une troisième section (12) extracorporelle, caractérisé en ce que la deuxième section (11) est composée d'une structure fibrillaire en matelas de fibres à pores ouverts, l'espacement moyen entre fibres, dans le matelas de fibres, augmentant de façon étagée ou continue, de l'intérieur vers l'extérieur.

2. Cathéter d'implantation transdermique selon la revendication 1, caractérisé en ce que la troisième section (12) est composée du même matériau que la deuxième section (11) et formée d'un seul tenant avec celle-ci, en présentant un revêtement (21) lisse fermé.

3. Cathéter d'implantation transdermique selon la revendication 2, caractérisé en ce qu'à la transition entre la première et la deuxième section (10,11) est monté un moyens auxiliaire tel qu'une rondelle, bille, gorge annulaire ou cannelure, permettant de réaliser une couture et une dilatation intérieure à l'emplacement de passage dans la cavité corporelle, en vue d'assurer une étanchéité.

4. Cathéter d'implantation transdermique selon l'une des revendications 1 à 3, caractérisé en ce que la deuxième section (11) présente des fils de renforcements (19) s'étendant hélicoïdalement.

5. Cathéter d'implantation transdermique selon l'une des revendications 1 à 4, caractérisé en ce que les fibres du matelas de fibres ont une épaisseur d'à peu près 4 à 5 mm.

6. Cathéter d'implantation transdermique selon l'une des revendications 1 à 5, caractérisé en ce que le matelas de fibres est en polyuréthane.

7. Cathéter d'implantation transdermique selon l'une des revendications 1 à 6, caractérisé en ce que la deuxième section. (11) est plus molle et flexible que la première section (10).

8. Cathéter d'implantation transdermique selon l'une des revendications 1 à 7, caractérisé en ce que la longueur de la deuxième section (11) atteint à peu près 5 cm.

9. Cathéter d'implantation transdermique selon l'une des revendications 1 à 8, caractérisé en ce que l'espacement moyen entre fibres dans le matelas de fibres augmente de 40 µm à 600 µm, en allant de l'intérieur vers l'extérieur.

## Claims

1. A transcutaneous implantation catheter for implantation through a tunneling zone into a body cavity, consisting of a hose with an intracorporal first section (10) of flexible solid plastic material, a second section (11) for implantation into the tunnel tissue and an extracorporal third section (12),
characterized in that
said second section (11) consists of a non-woven fiber fabric with a fibrillary open-pore structure, the mean fiber spacing in said fiber fabric increasing either stepwise or continuously from the inside to the outside.

2. The transcutaneous implantation catheter of claim 1, characterized in that said third section (12) is of the same material as said second section (11) to which it is integrally formed, and that said third section has a closed smooth sheathing (21).

3. The transcutaneous implantation catheter of claim 1 or 2, characterized in that an auxiliary means like a disc, a ball or an annular groove is provided at the transition between said first and second sections (10, 11), which allows the suturing and the growing of tissue onto passage into the body cavity.

4. The transcutaneous implantation catheter of one of claims 1 - 3, characterized in that said second section (11) has a helically extending reinforcing thread (19).

5. The transcutaneous implantation catheter of one of claims 1 - 4, characterized in that the fibers of said fiber fabric have a thickness of about 4 to 5 µm.

6. The transcutaneous implantation catheter of one of claims 1 - 5, characterized in that the fiber fabric is made of polyurethane.

7. The transcutaneous implantation catheter of one of claims 1 - 6, characterized in that said second section (11) is softer and more flexible than said first section (10).

8. The transcutaneous implantation catheter of one of claims 1 - 7, characterized in that the length of said second section (11) is about 5 cm.

9. The transcutaneous implantation catheter of one of claims 1 - 8, characterized in that the mean fiber spacing in said fiber fabric increases from the inside to the outside in a range from about 40 µm to about 600 µm.
